# EUROPÄISCHE PATENTSCHRIFT

(11) **EP 0 353 645 B1**
(45) Veröffentlichungstag und Bekanntmachung des Hinweises auf die Patenterteilung: **19.10.1994**
(21) Anmeldenummer: 89113945.3
(22) Anmeldetag: 28.07.1989
(51) Int. Cl.: C07D 493/04, C08G 73/10

(54) **Verfahren zur Herstellung von hochreinem 5,5-[2,2,2-trifluor-1-(trifluormethyl)-ethyliden] bis-1,3-isobenzofurandion**
Process for preparing high-purity 5,5-[2,2,2-trifluoro-1-(trifluoromethyl)-ethylidene] bis-1,3-isobenzofuran dione
Procédé de préparation de 5,5-[2,2,2-trifluoro-1-(trifluorométhyle)-éthylidène] bis-1,3-isobenzofuranne-dione de la haute pureté

(30) Priorität: 04.08.1988 DE 3826572
(43) Veröffentlichungstag der Anmeldung: 07.02.1990
(73) Patentinhaber: HOECHST AKTIENGESELLSCHAFT, 65926 Frankfurt am Main (DE)
(72) Erfinder: Röhrscheid, Freimund, Dr., D-6233 Kelkheim (Taunus) (DE)

(56) Entgegenhaltungen:
- EP-A- 0 317 884
- GB-A- 1 253 607
- US-A- 4 016 180

## Beschreibung

Die Erfindung betrifft ein Verfahren zur Herstellung von hochreinem "6F-DA" durch eine mehrstufige Behandlung der rohen Verbindungen. Der Metallionengehalt der behandelten Verbindungen wird erheblich erniedrigt. Verwendung findet das Anhydrid nach der Polymerisation mit einem aromatischen Diamin in der Mikroelektronik.
5,5′-[2,2,2-Trifluor-1-(trifluormethyl)-ethyliden] bis -1,3-isobenzofurandion, auch bekannt als 2,2-Bis-(3,4-dicarboxyphenyl) hexafluorpropan-dianhydrid, im nachfolgenden 6F-Dianhydrid oder abgekürzt "6F-DA" genannt, wird hauptsächlich für die Polykondensation mit aromatischen Diaminen zu Polyimiden verwendet. Diese Polyimide sind besonders für technisch anspruchsvolle Zwecke geeignet, z. B. für thermisch hoch belastbare Überzüge im Flugzeugbau oder wegen ihrer sehr niedrigen Dielektrizitätskonstanten in der Mikroelektronik. Dieses optimale Eigenschaftsspektrum der trifluormethylgruppenhaltigen Polyimide läßt sich jedoch nur durch Verwendung von hochreinem 6F-Dianhydrid erreichen.

Wenn also 6F-Dianhydrid durch Verbindungen wie 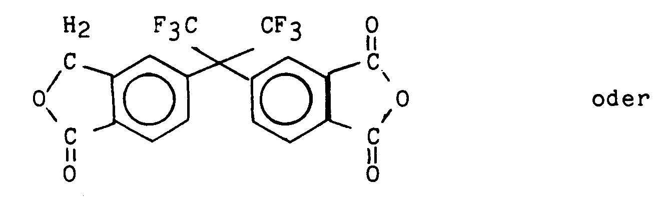 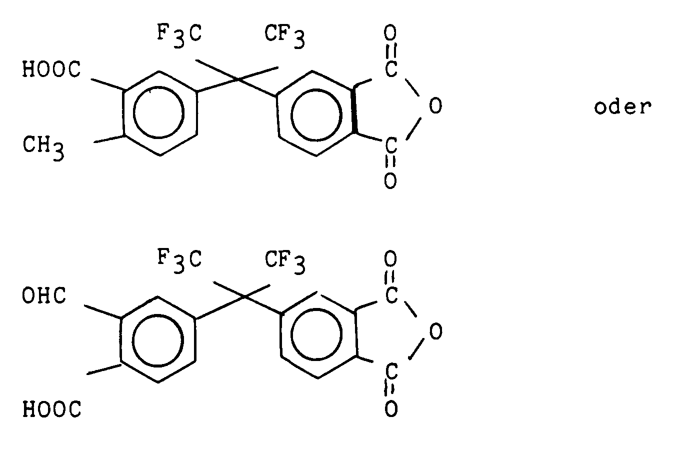
die aus dem Herstellungsprozeß stammen, verunreinigt ist, führt das entweder zum Abbruch der Polykondensation oder zu einer Schwachstelle in der Polyimidkette, wodurch die mechanische Festigkeit und die thermische Beständigkeit verringert werden. Für die Verwendung in der Mikroelektronik ist es außerdem erforderlich, daß das verwendete 6F-Dianhydrid einen sehr niedrigen Metallgehalt (Fe, Co, Mn, Ni, Na etc.) von hochstens 1 ppm besitzt.

Es bestand daher die Aufgabe, hochreines 6F-Dianhydrid zur Verfügung zu stellen, das mit aromatischen Diaminen polymerisiert, als Polyimid bevorzugt in der Mikroelektronik Verwendung finden kann.

Dixylylhexafluorpropan (DX-F6) und seine Oxidation mit Kaliumpermanganat in einer Mischung aus Pyridin und Wasser zum Kaliumsalz des 2,2-Bis-(3,4-dicarboxyphenyl)-hexafluorpropans (6F-Tetracarbonsäure) sowie die Herstellung des entsprechenden 6F-Dianhydrids wird in der US-A 3.310.573 beschrieben. Die Art der Oxidation benötigt einen hohen Chemikalienbedarf, die Isolierung der Tetracarbonsäure ist sehr umständlich, und das verwendete Lösungsmittelsystem sowie das Manganoxid müssen aufgearbeitet werden. Die Reinigung des 6F-Dianhydrids erfolgt durch einfache Sublimation. Über den Reinheitsgrad ist nichts ausgesagt worden.

Gelöst wurde die Aufgabe durch ein mehrstufiges Verfahren, bei dem eine unreine 6F-Tetracarbonsäure, wie sie beispielsweise durch Oxidation von Dixylylhexafluorpropan nach einem der bekannten Verfahrensweisen mit KMnO₄, Salpetersäure oder Luftsauerstoff anfällt, in mehreren Schritten von den Verunreinigungen befreit wird, wobei die wirksamen Schritte erst in ihrer Kombination die volle Breite der verschiedenen Arten von Verunreinigungen erfassen.

Die Erfindung betrifft daher ein Verfahren zur Herstellung von hochreinem 5,5′-[2,2,2-Trifluor-1-(trifluormethyl)-ethyliden] bis-1,3-isobenzofurandion (6F-Dianhydrid = 6F-DA), bei dem eine konzentrierte Lösung von 6F-Tetracarbonsäure in Wasser oder in verdünnter Essigsäure folgenden Schritten unterworfen wird:
a) Behandlung der Lösung mit Adsorbentien und/oder Filterhilfsstoffen und anschließende Filtration,
b) Behandlung des Filtrats a) mit Ionenaustauscher
c) Abkühlung der nach b) behandelten Lösung zur Bildung von reiner 6F-Tetracarbonsäure,
d) Filtration der 6F-Tetracarbonsäure und
e) Umwandlung der 6F-Tetracarbonsäure in das 6F-Dianhydrid, so daß
die erhaltene Verbindung eine Reinheit von ≧ 99,5 % bei einem Gesamtmetallionengehalt von ≦ 1 ppm aufweist. Diese Verfahrensschritte a) bis e) sind in sich noch einmal speziell ausgestaltet und werden nachfolgend näher behandelt.

Nach einer noch unveröffentlichten, besonders günstigen Verfahrensweise (Deutsche Patentanmeldung P 37 39 800.8) wird Dixylylhexafluorpropan in Gegenwart von Co-, Mn-, Ce-und Br-Ionen in Eisessig mit Luftsauerstoff zur 6F-Tetracarbonsäure oxidiert. Durch Zugabe von Acetanhydrid zur oxidierten und entwässerten Reaktionslösung kristallisiert 6F-Dianhydrid aus, das nach Waschen und Trocknen mit 96 % Ausbeute in einer Reinheit von 94 - 96 % vorliegt, die für die oben genannten Zwecke aber nicht ausreicht.

Die direkte Isolierung der 6F-Tetracarbonsäure aus der Reaktionslösung ist nicht möglich, weil sie oft erst nach Tagen und dann nur unvollständig in schlecht filtrierbarer Form auskristallisiert.

Um zu einer konzentrierten Lösung von 6F-Tetracarbonsäure in Wasser oder in Essigsäure-Wasser-Gemischen zu gelangen, kann man 1) direkt von der Oxidationslösung, wie vorstehend beschrieben, ausgehen oder aber 2) den Weg über das 6F-Dianhydrid wählen, indem man dieses hydrolysiert.
1) Die Oxydationslösung wird bis zu einer Sumpftemperatur von 130 - 160° C, vorzugsweise 140 - 150° C, besonders bevorzugt 145° C, eingedampft, dann wird unter Inertgasdruck von ca. 6 bar bei 135 - 140° C soviel Wasser zudosiert, daß die Essigsäurekonzentration 2 - 30, vorzugsweise 5 - 20, insbesondere 8 - 12 Gew-% beträgt. Bei dieser Verfahrensweise war es überraschend, daß beim Eindampfen der Reaktionslösung keine Kristallisation wie bei anderen Polycarbonsäuren eintrat, sondern daß bei 145° C Sumpftemperatur eine rührbare Schmelze mit niedrigem Essigsäuregehalt vorlag.
2) 6F-Dianhydrid ist mit siedendem Wasser nur sehr langsam im Laufe von Tagen zu hydrolysieren. Es wurde überraschend gefunden, daß die Hydrolyse des 6F-Dianhydrids in weniger als einer halben Stunde beendet ist, wenn man dem Wasser Essigsäure in den vorstehend ausgeführten Mengen zufügt. Besonders vorteilhaft ist hier der Einsatz von 7 - 8 % Essigsäure.

Die Lösung von 6F-Tetracarbonsäure in verdünnter wäßriger Essigsäure besitzt eine Trübung, die aus feinstverteiltem Eisensalz der 6F-Tetracarbonsäure besteht, und die auch durch feinporige Filter nicht zurückgehalten wird. Durch Zusatz von Adsorbentien und/oder Filterhilfsstoffen im Verfahrensschritt a) wie Aktivkohle, Kieselgur oder durch Kombination von Aktivkohle und Kieselgur wird diese Trübung adsorbiert und abfiltrierbar. Die Menge der zugesetzten Mittel richtet sich nach dem Verunreinigungsgrad. Im allgemeinen ist der Zusatz von 2 bis 10 g pro Liter Lösung ausreichend. In der Stufe b wird die klare Lösung a) über einen stark sauren Ionenaustauscher, der vorzugsweise aus einer Polystyrolsulfonsäure besteht, geleitet. Es ist ebenfalls bevorzugt, diese Behandlung bei erhöhter Temperatur, beispielsweise bei 70 bis 90° C durchzuführen. Die Metallionen werden dadurch nahezu quantitativ entfernt. Überraschenderweise steigt auch die Ausbeute an 6F-Tetracarbonsäure um einen Betrag, der etwa der Val-Menge der Metallionen entspricht.

Zur Kristallisation der 6F-Tetracarbonsäure wird die Lösung b) auf 10 - 25° C, vorzugsweise auf 18 - 22° C, abgekühlt. Die blättchenförmigen Kristalle werden filtriert und mit Wasser oder verdünnter wäßriger Essigsäure gewaschen. Im Filtrat befinden sich hauptsächlich solche Carbonsäuren, die auch nach dem Eindampfen nicht kristallisieren. Die Filtration verläuft in vielen Fällen sehr langsam. Die Filtrationszeit kann überraschenderweise verkürzt werden, wenn man aus verdünnter wäßriger Essigsäure mit vorzugsweise 8 - 12 % Essigsäure im Wasser kristallisierte. Eine Verkürzung der Filtrationszeit wird auch dadurch erreicht, daß man, bezogen auf das wäßrige Lösemittel, 0,1 bis 2, vorzugsweise 0,2 bis 0,6 Gew.-%, eines inerten Kohlenwasserstoffes, vorzugsweise Toluol, Äthylbenzol oder die verschiedenen Xylole hinzufügt.

Der Filterkuchen aus 6F-Tetracarbonsäure, der einen Gesamtmetallionengehalt von ≦ 1 ppm aufweist, kann getrocknet und durch Erhitzen unter vermindertem Druck auf 175 bis 220° C, vorzugsweise 180 - 190° C, zum 6F-Dianhydrid dehydratisiert werden.

Es ist möglich, die Reinheit des 6F-Dianhydrids weiter, d. h. auf ≧ 99,5 % zu verbessern, indem die Bildung des 6F-Dianhydrids in einem Kohlenwasserstoff, vorzugsweise in einem aromatischen Kohlenwasserstoff, vorgenommen wird. Eine besonders einfache Verfahrensweise besteht darin, den wasserfeuchten Filterkuchen der 6F-Tetracarbonsäure in einen inerten Kohlenwasserstoff zu suspendieren und das Wasser azeotrop abzudestillieren. Die dadurch gebildete Suspension von wasserfreier 6F-Tetracarbonsäure kann auf zwei Wegen in das 6F-Dianhydrid überführt werden.
a) thermisch. Durch Erhitzen auf oberhalb 140°C wird aus der Dicarbonsäure der Anhydridring durch Wasserabspaltung gebildet. Eine schnelle Anhydridbildung wird vorzugsweise oberhalb 170°C, besonders bevorzugt bei 180 - 220°C, durchgeführt. Geeignet sind Lösemittel mit hohem Siedepunkt wie Diphenylether oder Tetrahydronaphthalin, oder es werden gegebenenfalls tiefer siedende Lösemittel wie Toluol oder o-Xylol bei 180-220°C unter Inertgasdruck verwendet. Sobald kein Wasser mehr übergeht, läßt man die Lösung unter Rühren abkühlen.
b) chemisch. Zur wasserfreien Suspension von 6F-Tetracarbonsäure in dem Kohlenwasserstoff wird so viel Acetanhydrid gegeben, daß ein Überschuß vorliegt, vorzugsweise 0,5 - 25 mol-% Acetanhydrid, bezogen auf die theoretisch mögliche Ausbeute von 6F-Dianhydrid. Das 6F-Dianhydrid bildet schöne, schwere Kristalle, von denen ein Teil erst beim Abkühlen auskristallisiert. Verunreinigungen, z. B. solche mit der Formel 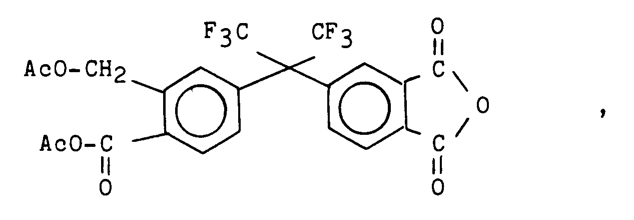 Ac = CH₃CO-
   bleiben in Lösung.
   Nach dem Absaugen werden die Kristalle mit einem Kohlenwasserstoff gewaschen und getrocknet. Für die chemische Bildung von 6F-Dianhydrid werden vorzugsweise aromatische Kohlenwasserstoffe mit einem mittleren Siedebereich von 110 - 150°C verwendet, z.B. Toluol, Ethylbenzol und die verschiedenen Xylole. Mit diesen Kohlenwasserstoffen läßt sich eine genügend hohe Reaktionstemperatur erreichen, andererseits liegen ihre Siedepunkte noch tief genug, daß sie leicht durch einfache Trocknung unter vermindertem Druck aus dem abgesaugten Kristallisat des 6F-Dianhydrids entfernt werden können.

### Beispiel

### 1. Herstellung einer Lösung von 6F-Tetracarbonsäure 2,2-Bis-(3,4-Dicarboxyphenyl)-hexafluorpropan-(6F-Tetracarbonsäure)

In einem 1 l-Glasautoklaven, der mit Dosierpumpe, Thermometer, Rührer und Rückflußkühler ausgestattet war, wurde eine Lösung von 2,5 g Kobaltacetattetrahydrat, 2,45 g Manganacetattetrahydrat und 0,44 g Bromwasserstoff in 310 ml Eisessig vorgelegt. Parallel dazu wurde eine Lösung von 180,2 g Dixylylhexafluorpropan (DX-6F)in einem Gemisch von 102 g Acetanhydrid und 60 g Eisessig in einer Dosiereinrichtung bereitgestellt. Der Autoklav wurde durch Einleiten von Sauerstoff unter einen Gesamtdruck von 7,5 bar gesetzt, ein Abgaswert von 30 Nl/h eingestellt und der Inhalt erhitzt.

Bei ca. 160°C wurde mit der Zudosierung von Dixylylhexafluorpropan begonnen und innerhalb von 100 Minuten die Gesamtmenge hinzugefügt. Die Temperatur der exothermen Reaktion wurde bei 170-175°C gehalten und der Ansatz nach Beendigung der Dosierung für eine weitere Stunde bei 175°C durch Heizen gehalten. Von der Reaktionsmischung (ca. 790 bis 800 g) wurden bei Normaldruck ca. 500 g Essigsäure-Wasser-Gemisch abdestilliert. Sobald die Temperatur des Rückstandes auf 145°C gestiegen war, wurde der Autoklav durch Einleiten von Stickstoff auf einen Druck von 4 bar gesetzt, 500 g destilliertes Wasser zugegeben und die Mischung für eine Stunde bei 145°C gehalten. Dann wurde auf etwa 90°C abgekühlt und die Lösung entnommen.

### 2. Klären und Filtration (Stufe a)

Zur heißen Lösung (90°C), die eine leichte Trübung hatte, wurde die Suspension von 4 g Kieselgur in 12 g Wasser gegeben. Die Mischung wurde 15 Minuten bei 90°C gerührt und heiß über ein Saugfilter gegeben. Die ersten 100 ml des Filtrats werden zurückgegeben und nochmals filtriert.

### 3. Entfernen der Metallionen (Stufe b)

Das klare Filtrat (ca. 800 g ≙ 675 ml) mit 8% Essigsäuregehalt wurde in ein beheizbares, hochgehängtes 1 l-Gefäß gefüllt und auf 80°C erhitzt. Aus diesem wurde die Lösung auf eine auf 80°C beheizte senkrechte Austauschersäule (Länge 100 cm, Durchmesser 2 cm) geleitet, die 80 cm hoch mit frisch aktiviertem, stark saurem Ionenaustauscher (Polystyrolsulfonsäure, 2,1 Mol/l,®Lewatit S 100 der Fa. Bayer AG, Leverkusen) gefüllt war. Durchlaufdauer 2 Std.. Zum Schluß wurde die Säule mit 100 ml Wasser gespült. 775 g Eluat, Metallionengehalt: < 1 mg Co, Mn pro Liter Lösung.

### 4. Kristallisation und Wäsche der 6F-Tetracarbonsäure (Stufen c und d)

Die Kristallisation wurde in einem zylindrischen Gefäß mit Ankerrührer durchgeführt. Dem heißen Eluat (775 ml) wurden 3 ml o-Xylol zugefügt. Es wurde auf 21°C abgekühlt. Der entstandene dicke Brei von Kristallblättchen wurde abgesaugt. Der Filterkuchen wurde durch 12malige Zugabe von je 25 ml destilliertem Wasser gewaschen und zum Schluß für ca. 30 Minuten trockengesaugt. Ausbeute: 400 g wasserfeuchtes Produkt.

### 5. Bildung des 6F-Dianhydrids (Stufe e)

Die wasserfeuchte 6F-Tetracarbonsäure (400 g) wurde in einem Kolben, der mit Rührer und Wasserabscheider ausgerüstet war, mit 450 ml o-Xylol suspendiert. Der Ansatz wurde unter starkem Rühren erhitzt, und es destillierten etwa 180 g Wasser ab. Zum Schluß stieg die Innentemperatur auf 142°C und es destillierte nur noch o-Xylol. Im Kolben verblieb eine Kristallsuspension von wasserfreier 6F-Tetracarbonsäure. Innerhalb von 2 Stunden wurde Essigsäureanhydrid (118,8 g) in die siedende Suspension eingetropft. Anschließend wurde noch weitere 3 Stunden am Rückfluß erhitzt. Die Konzentration an Essigsäureanhydrid betrug am Ende ca. 4,5 %. Die Suspension wurde unter Rühren im Laufe von 3 Stunden auf 30°C abgekühlt, anschließend abgenutscht und viermal mit je 35 ml o-Xylol gewaschen. Nach der letzten Wäsche wurde scharf abgesaugt. Die ersten Filtrate waren gelb, das letzte farblos. Der schön kristalline Filterkuchen wurde 6 Stunden bei 100°C und 65 mbar getrocknet.

| | |
|---|---|
| Ausbeute | 195 g (ca. 88 % d. Th., bez. auf DX-F6) |
| Fp | 244 - 245°C |
| Reinheit | 99,9 % |
| Metallgehalt | 0,5 ppm (Summe aller Metallionen) |

## Patentansprüche

1. Verfahren zur Herstellung von hochreinem 5,5′-[2,2,2-Trifluor-1-(trifluormethyl)-ethyliden]bis-1,3-isobenzofurandion (6F-Dianhydrid = 6F-DA), dadurch gekennzeichnet, daß eine konzentrierte Lösung von 6F-Tetracarbonsäure in Wasser oder in verdünnter Essigsäure folgenden Schritten unterworfen wird:
a) Behandlung der Lösung mit Adsorbentien und/oder Filterhilfsstoffen und anschließende Filtration,
b) Behandlung des Filtrats a) mit Ionenaustauscher,
c) Abkühlung der nach b) behandelten Lösung zur Bildung von reiner 6F-Tetracarbonsäure,
d) Filtration der 6F-Tetracarbonsäure und
e) Umwandlung der 6F-Tetracarbonsäure in das 6F-Dianhydrid, so daß
die erhaltene Verbindung eine Reinheit von ≧ 99,5 % bei einem Gesamtmetallionengehalt von ≦ 1 ppm aufweist.

2. Verfahren nach Anspruch 1, dadurch gekennzeichnet, daß als konzentrierte Lösung eine Reaktionslösung der Herstellung von 6F-Tetracarbonsäure oder ein Hydrolysat von 6F-DA eingesetzt wird.

3. Verfahren nach Anspruch 2, dadurch gekennzeichnet, daß die konzentrierte Lösung eine Essigsäurekonzentration von 2 bis 30, vorzugsweise 5 bis 20, insbesondere 8 bis 12 Gew.-% aufweist.

4. Verfahren nach einem oder mehreren der Ansprüche 1 bis 3, dadurch gekennzeichnet, daß in der Stufe a) Aktivkohle, Kieselgur oder ein Gemisch von beiden eingesetzt wird.

5. Verfahren nach einem oder mehreren der Ansprüche 1 bis 4, dadurch gekennzeichnet, daß kolloidale Metallsalze in der Stufe a) entfernt werden.

6. Verfahren nach einem oder mehreren der Ansprüche 1 bis 5, dadurch gekennzeichnet, daß der Ionenaustauscher stark sauer ist und vorzugsweise aus einer Polystyrolsulfonsäure besteht.

7. Verfahren nach einem oder mehreren der Ansprüche 1 bis 6, dadurch gekennzeichnet, daß die Behandlung in Stufe b) bei erhöhter Temperatur, vorzugsweise bei 70 - 90°C durchgeführt wird.

8. Verfahren nach einem oder mehreren der Ansprüche 1 bis 7, dadurch gekennzeichnet, daß die Temperatur der aus Stufe b) erhaltenen Lösung auf 10 bis 25°C, insbesondere auf 18 - 22°C erniedrigt wird.

9. Verfahren nach einem oder mehreren der Ansprüche 1 bis 8, dadurch gekennzeichnet, daß vor der Abkühlung der Lösung 0,1 bis 2, vorzugsweise 0,2 - 0,6 Gew.-%, bezogen auf das wässerige Lösungsmittel, eines inerten Kohlenwasserstoffes unter Rühren hinzugegeben wird.

10. Verfahren nach einem oder mehreren der Ansprüche 1 bis 9, dadurch gekennzeichnet, daß ein aromatischer Kohlenwasserstoff, vorzugsweise Toluol, Äthylbenzol oder die verschiedenen Xylole, eingesetzt wird.

11. Verfahren nach einem oder mehreren der Ansprüche 1 bis 10, dadurch gekennzeichnet, daß e) die Bildung des 6F-DA aus der gereinigten 6F-Tetracarbonsäure nach den Verfahren vorgenommen wird:
e1) durch Erhitzen des getrockneten Filterkuchens der Säure unter vermindertem Druck auf 175-220, vorzugsweise 180-190°C,
e2) durch Erhitzen einer wasserfreien Suspension der Säure in einem inerten Lösungsmittel bei einer Temperatur oberhalb 140°C, vorzugsweise oberhalb 170°C, insbesondere bei 180-220°C, gegebenenfalls unter Inertgasdruck, oder
e3) durch Umsetzung einer wasserfreien Suspension der Säure in einem inerten Lösungsmittel mit Acetanhydrid, vorzugsweise bei 110-140°C,
wobei bei den Verfahren e2) und e3) die erhaltenen Kristalle mit einem Kohlenwasserstoff gewaschen und anschließend getrocknet werden.

12. Verfahren nach Anspruch 11, dadurch gekennzeichnet, daß bei dem Verfahren e2) Diphenylether, Tetrahydronaphthalin, Toluol oder o-Xylol als Lösungsmittel verwendet werden.

13. Verwendung des nach dem Verfahren gemäß einem oder mehreren der Ansprüche 1 bis 12 hergestellten 6F-Dianhydrids zur Herstellung von Polyimiden.

14. Verwendung der Polyimide nach Anspruch 13 in der Mikroelektronik.

## Claims

1. A process for the preparation of high-purity 5,5'-(2,2,2-trifluoro-1-trifluoromethylethylidene)bis-1,3-isobenzofurandione (6F dianhydride = 6F-DA), which comprises subjecting a concentrated solution of 6F tetracarboxylic acid in water or in dilute acetic acid to the following steps:
a) treatment of the solution with adsorbents and/or filtering aids, followed by filtration,
b) treatment of filtrate a) with ion exchanger,
c) cooling of the solution treated according to b) to form pure 6F tetracarboxylic acid,
d) filtration of 6F tetracarboxylic acid and
e) conversion of 6F tetracarboxylic acid to 6F dianhydride,
as a result of which the compound obtained has a purity of ≧ 99.5% at a total metal ion content of ≦ 1 ppm.

2. The process as claimed in claim 1, wherein the concentrated solution used is a reaction solution from the preparation of 6F tetracarboxylic acid or a hydrolysis product of 6F-DA.

3. The process as claimed in claim 2, wherein the concentrated solution has an acetic acid concentration of 2 to 30, preferably 5 to 20, in particular 8 to 12 % by weight.

4. The process as claimed in one or more of claims 1 to 3, wherein in step a) activated carbon, kieselguhr or a mixture of both is used.

5. The process as claimed in one or more of claims 1 to 4, wherein colloidal metal salts are removed in step a).

6. The process as claimed in one or more of claims 1 to 5, wherein the ion exchanger is strongly acidic and preferably consists of a polystyrenesulfonic acid.

7. The process as claimed in one or more of claims 1 to 6, wherein the treatment in step b) is carried out at elevated temperature, preferably at 70 - 90°C.

8. The process as claimed in one or more of claims 1 to 7, wherein the temperature of the solution obtained from step b) is reduced to 10 to 25°C, in particular to 18 - 22°C.

9. The process as claimed in one or more of claims 1 to 8, wherein, before the solution is cooled, 0.1 to 2, preferably 0.2 - 0.6, % by weight, relative to the aqueous solvent, of an inert hydrocarbon is added with stirring.

10. The process as claimed in one or more of claims 1 to 9, wherein an aromatic hydrocarbon, preferably toluene, ethylbenzene or the various xylenes, is used.

11. The process as claimed in one or more of claims 1 to 10, wherein e) the formation of 6F-DA from the purified 6F tetracarboxylic acid is carried out by the process:
e1) heating the dried filter cake of the acid under reduced pressure to 175 - 220, preferably 180 - 190, °C,
e2) heating an anhydrous suspension of the acid in an inert solvent at a temperature above 140°C, preferably above 170°C, in particular 180 - 220°C, if appropriate under inert gas pressure, or
e3) reacting an anhydrous suspension of the acid in an inert solvent with acetic anhydride, preferably at 110 - 140°C,
the crystals obtained in processes e2) and e3) being washed with a hydrocarbon and subsequently dried.

12. The process as claimed in claim 11, wherein in process e2) diphenyl ether, tetrahydronaphthalene, toluene or o-xylene is used as the solvent.

13. Use of the 6F dianhydride prepared by the process as claimed in one or more of claims 1 to 12 for the preparation of polyimides.

14. Use of the polyimides as claimed in claim 13 in microelectronics.

## Revendications

1. Procédé de préparation de la 5,5'-[2,2,2-trifluoro-1-(trifluorométhyl)-éthylidène]-bis-1,3-isobenzofuranne-dione de haute pureté (6F-dianhydride = 6F-DA), caractérisé en ce qu'on soumet une solution concentrée d'acide 6F-tétracarboxylique dans de l'eau ou dans de l'acide acétique dilué, aux étapes suivantes :
(a) Traitement de la solution par des adsorbants et/ou par des adjuvants de filtration, puis filtration;
(b) Traitement du filtrat (a) par de l'échangeur d'ions;
(c) Refroidissement de la solution traitée selon (b) pour former de l'acide 6F-tétracarboxylique pur;
(d) Filtration de l'acide 6F-tétracarboxylique et,
(e) Transformation de l'acide 6F-tétracarboxylique en le 6F-dianhydride
de sorte que le composé obtenu présente une pureté égale ou supérieure à 99,5 % avec une teneur totale en des ions de métaux inférieure ou égale à 1 ppm.

2. Procédé selon la revendication 1, caractérisé en ce qu'on utilise comine solution concentrée une solution de réaction provenant de la préparation de l'acide 6F-tétracarboxylique ou un hydrolysat du "6F-DA" .

3. Procédé selon la revendication 1, caractérisé en ce que la solution concentrée présente une concentration d'acide acétique de 2 à 30, avantageusement de 5 à 20, notamment de 8 à 12 % en poids.

4. Procédé selon une ou plusieurs des revendications 1 à 3, caractérisé en ce que, dans l'étape (a), on utilise du charbon actif, du Kieselghur ou un mélange des deux.

5. Procédé selon une ou plusieurs des revendications 1 à 4, caractérisé en ce qu'on élimine dans l'étape (a)des sels colloïdaux de métaux.

6. Procédé selon une ou plusieurs des revendications 1 à 5, caractérisé en ce que l'échangeur d'ions est fortement acide, et il consiste avantageusement en un acide polystyrène sulfonique.

7. Procédé selon une ou plusieurs des revendications 1 à 6, caractérisé en ce que le traitement dans l'étape (b) est conduit à une température élevée, se situant avantageusement à 70-90°C.

8. Procédé selon une ou plusieurs des revendications 1 à 7, caractérisé en ce que la température de la solution obtenue de l'étape (b)est abaissée à 10 jusqu'à 25°C, notamment à 18-22°C.

9. Procédé selon une ou plusieurs des revendications 1 à 8, caractérisé en ce que, avant le refroidissement de la solution, on y introduit sous agitation 0,1 à 2, avantageusement 0,2 à 0,6 % en poids, par rapport au solvant aqueux, d'un hydrocarbure inerte.

10. Procédé selon une ou plusieurs des revendications 1 à 9, caractérisé en ce qu'on utilise un hydrocarbure aromatique, avantageusement du toluène, de l'éthyl benzène ou les divers xylènes.

11. Procédé selon une ou plusieurs des revendications 1 à 10, caractérisé en ce que (e), la formation du "6F-DA" à partir de l'acide 6F-tétracarboxylique purifié est entreprise selon le procédé suivant :
(e1) par chauffage du gâteau séché de la filtration de l'acide sous presison réduite à 175-220°C, avantageusement 180 à 190C;
(e2) par chauffage d'une solution anhydre de l'acide dans un solvant inerte, à une température supérieure à 140°C, avantageusement supérieure à 170°C, en particulier à 180-220°C, éventuellement sous pression de gaz inerte, ou bien
(e3) par réaction d'une suspension anhydre de l'acide dans un solvant inerte avec de l'anhydride acétique, avantageusement à 110-140°C,
et, dans les procédés (e2) et (e3), les cristaux obtenus sont lavés avec un hydrocarbure puis séchés.

12. Procédé selon la revendication 11, caractérisé en ce que, dans le procédé (2), on utilise de l'éther oxyde de diphényle, du tétrahydronaphtalène, du toluène ou de l'o-xylène comme solvant.

13. Utilisation du 6F-dianhydride, préparé selon le procédé selon une ou plusieurs des revendications 1 à 12, pour produire des polyimides.

14. Utilisation des polyimides selon la revendication 13 en microélectronique.
